# EUROPEAN PATENT APPLICATION

(11) **EP 4 053 163 A1**
(43) Date of publication of application: **07.09.2022**
(21) Application number: 21161100.9
(22) Date of filing: 05.03.2021
(51) Int. Cl.: C07K 17/08, C12N 5/00, C12N 5/071, C08F 8/00, C08L 33/00

(54) **CELL ATTACHMENT MATERIALS, DEVICES AND USES THEREOF**

(71) Applicant: FaCellitate GmbH, 68169 Mannheim (DE)
(72) Inventor: KAISER, Nadine, 67056 Ludwigshafen (DE); MENTZEL, Tobias, 67117 Limburgerhof (DE)
(74) Representative: Eisenführ Speiser

(57) **Abstract**

The present invention is concerned with materials and methods for mediating eukaryotic cell attachment to rigid surfaces. In particular, the invention is concerned with coatings for forming cell attachment surfaces, preferably for cell culture devices, microfluidic devices, biosensors or implants. The invention correspondingly provides polymer conjugates for such coatings, methods of polymer conjugate formation, methods of polymer conjugate application and uses of such polymer conjugates. The invention also provides surfaces and devices at least partly coated with such polymer conjugates.

SEQUENCE LISTING

<110>  BASF

<120>  TITLE CELL ATTACHMENT MATERIALS, DEVICES AND USES THEREOF

<130>  PF 210190

<160>  42

<210> 1
<211> 9
<212> PRT
<213> Artificial sequence

<220>
<223> NOTE: artificial peptide for polymer conjugates

<400> 1
Cys Trp Gly Gly Arg Gly Asp Ser Pro
1               5

<210> 2
<211> 5
<212> PRT
<213> Artificial sequence

<220>
<223> NOTE: artificial peptide for polymer conjugates

<220>
<223> NOTE: peptide is cyclic

<220>
<221> SITE
<222> (4)..(4)
<223> Tyr at position 4 is D-Tyrosin

<400> 2
Arg Gly Asp Tyr Cys
1               5

<210> 3
<211> 17
<212> PRT
<213> Artificial sequence

<220>
<223> NOTE: artificial peptide for polymer conjugates

<400> 3
Gly Cys Trp Gly Gly Pro Gln Val Thr Arg Gly Asp Val Phe Thr Met
1               5                   10                  15
Pro

<210> 4
<211> 17
<212> PRT
<213> Artificial sequence

<220>
<223> NOTE: artificial peptide for polymer conjugates

<400> 4
Gly Cys Trp Gly Gly Gln Pro Pro Arg Ala Arg Ile Thr Gly Tyr Ile
1               5                   10                  15
Ile

<210> 5
<211> 18
<212> PRT
<213> Artificial sequence

<220>
<223> NOTE: artificial peptide for polymer conjugates

<400> 5
Gly Cys Trp Trp Gly Gly Asn Gly Glu Pro Arg Gly Asp Thr Tyr Arg
1               5                   10                  15
Ala Tyr

<210> 6
<211> 19
<212> PRT
<213> Artificial sequence

<220>
<223> NOTE: artificial peptide for polymer conjugates

<400> 6
Gly Cys Trp Gly Arg Lys Lys Gly Arg Lys Lys Gly Arg Lys Lys Gly
1               5                   10                  15
Arg Lys Lys

<210> 7
<211> 19
<212> PRT
<213> Artificial sequence

<220>
<223> NOTE: artificial peptide for polymer conjugates

<400> 7
Gly Cys Trp Gly Lys Lys Arg Gly Lys Lys Arg Gly Lys Lys Arg Gly
1               5                   10                  15
Lys Lys Arg

<210> 8
<211> 15
<212> PRT
<213> Artificial sequence

<220>
<223> NOTE: artificial peptide for polymer conjugates

<400> 8
Gly Cys Trp Gly Arg Arg Arg Ala Arg Gly Lys Lys Lys Ala Lys
1               5                   10                  15

<210> 9
<211> 4
<212> PRT
<213> Artificial sequence

<220>
<223> NOTE: artificial peptide for polymer conjugates

<400> 9
Arg Gly Asp Ser
1

<210> 10
<211> 4
<212> PRT
<213> Artificial sequence

<220>
<223> NOTE: artificial peptide for polymer conjugates

<400> 10
Arg Gly Asp Thr
1

<210> 11
<211> 4
<212> PRT
<213> Artificial sequence

<220>
<223> NOTE: artificial peptide for polymer conjugates

<400> 11
Arg Gly Asp Val
1

<210> 12
<211> 4
<212> PRT
<213> Artificial sequence

<220>
<223> NOTE: artificial peptide for polymer conjugates

<400> 12
Gly Arg Gly Asp
1

<210> 13
<211> 5
<212> PRT
<213> Artificial sequence

<220>
<223> NOTE: artificial peptide for polymer conjugates

<400> 13
Gly Arg Gly Asp Gly
1               5

<210> 14
<211> 5
<212> PRT
<213> Artificial sequence

<220>
<223> NOTE: artificial peptide for polymer conjugates

<400> 14
Gly Arg Gly Asp Phe
1               5

<210> 15
<211> 5
<212> PRT
<213> Artificial sequence

<220>
<223> NOTE: artificial peptide for polymer conjugates

<400> 15
Gly Arg Gly Asp Ser
1               5

<210> 16
<211> 5
<212> PRT
<213> Artificial sequence

<220>
<223> NOTE: artificial peptide for polymer conjugates

<400> 16
Gly Arg Gly Asp Tyr
1               5

<210> 17
<211> 5
<212> PRT
<213> Artificial sequence

<220>
<223> NOTE: artificial peptide for polymer conjugates

<400> 17
Tyr Arg Gly Asp Gly
1               5

<210> 18
<211> 5
<212> PRT
<213> Artificial sequence

<220>
<223> NOTE: artificial peptide for polymer conjugates

<400> 18
Tyr Arg Gly Asp Ser
1               5

<210> 19
<211> 5
<212> PRT
<213> Artificial sequence

<220>
<223> NOTE: artificial peptide for polymer conjugates

<400> 19
Tyr Gly Arg Gly Asp

1                   5

<210> 20
<211> 5
<212> PRT
<213> Artificial sequence

<220>
<223> NOTE: artificial peptide for polymer conjugates

<400> 20
Arg Gly Asp Ser Gly
1                   5

<210> 21
<211> 5
<212> PRT
<213> Artificial sequence

<220>
<223> NOTE: artificial peptide for polymer conjugates

<400> 21
Arg Gly Asp Ser Pro
1                   5

<210> 22
<211> 5
<212> PRT
<213> Artificial sequence

<220>
<223> NOTE: artificial peptide for polymer conjugates

<400> 22
Arg Gly Asp Ser Tyr
1                   5

<210> 23
<211> 5
<212> PRT
<213> Artificial sequence

<220>
<223> NOTE: artificial peptide for polymer conjugates

<400> 23
Arg Gly Asp Val Tyr
1                   5

<210> 24
<211> 6
<212> PRT
<213> Artificial sequence

<220>
<223> NOTE: artificial peptide for polymer conjugates

<400> 24
Gly Arg Gly Asp Ser Pro
1                   5

<210> 25

<211> 6
<212> PRT
<213> Artificial sequence

<220>
<223> NOTE: artificial peptide for polymer conjugates

<400> 25
Gly Arg Gly Asp Ser Gly
1               5

<210> 26
<211> 6
<212> PRT
<213> Artificial sequence

<220>
<223> NOTE: artificial peptide for polymer conjugates

<400> 26
Gly Arg Gly Asp Ser Tyr
1               5

<210> 27
<211> 6
<212> PRT
<213> Artificial sequence

<220>
<223> NOTE: artificial peptide for polymer conjugates

<400> 27
Gly Arg Gly Asp Val Tyr
1               5

<210> 28
<211> 7
<212> PRT
<213> Artificial sequence

<220>
<223> NOTE: artificial peptide for polymer conjugates

<400> 28
Gly Arg Gly Asp Ser Pro Lys
1               5

<210> 29
<211> 8
<212> PRT
<213> Artificial sequence

<220>
<223> NOTE: artificial peptide for polymer conjugates

<400> 29
Cys Gly Arg Gly Asp Ser Pro Lys
1               5

<210> 30
<211> 7
<212> PRT
<213> Artificial sequence

<220>
<223> NOTE: artificial peptide for polymer conjugates

<400> 30
Cys Gly Arg Gly Asp Ser Tyr
1               5

<210> 31
<211> 5
<212> PRT
<213> Artificial sequence

<220>
<223> NOTE: artificial peptide for polymer conjugates

<220>
<221> SITE
<222> (4)..(4)
<223> Phe at position 4 is D-Phenylalanine

<400> 31
Arg Gly Asp Phe Lys
1               5

<210> 32
<211> 9
<212> PRT
<213> Artificial sequence

<220>
<223> NOTE: artificial peptide for polymer conjugates

<400> 32
Tyr Ala Val Thr Gly Arg Gly Asp Ser
1               5

<210> 33
<211> 14
<212> PRT
<213> Artificial sequence

<220>
<223> NOTE: artificial peptide for polymer conjugates

<400> 33
Cys Gly Gly Asn Gly Glu Pro Arg Gly Asp Tyr Arg Ala Tyr
1               5                   10

<210> 34
<211> 11
<212> PRT
<213> Artificial sequence

<220>
<223> NOTE: artificial peptide for polymer conjugates

<400> 34
Gly Cys Gly Tyr Gly Arg Gly Asp Ser Pro Gly
1               5                   10

<210> 35
<211> 10

<212> PRT
<213> Artificial sequence

<220>
<223> NOTE: artificial peptide for polymer conjugates

<400> 35
Arg Gly Asp Ser Pro Ala Ser Ser Lys Pro
1               5                   10

<210> 36
<211> 11
<212> PRT
<213> Artificial sequence

<220>
<223> NOTE: artificial peptide for polymer conjugates

<400> 36
Gly Arg Gly Asp Ser Pro Ala Ser Ser Lys Gly
1               5                   10

<210> 37
<211> 8
<212> PRT
<213> Artificial sequence

<220>
<223> NOTE: artificial peptide for polymer conjugates

<400> 37
Cys Trp Gly Gly Arg Gly Asp Ser
1               5

<210> 38
<211> 8
<212> PRT
<213> Artificial sequence

<220>
<223> NOTE: artificial peptide for polymer conjugates

<400> 38
Cys Trp Gly Gly Arg Gly Asp Thr
1               5

<210> 39
<211> 8
<212> PRT
<213> Artificial sequence

<220>
<223> NOTE: artificial peptide for polymer conjugates

<400> 39
Cys Trp Gly Gly Arg Gly Asp Val
1               5

<210> 40
<211> 9
<212> PRT
<213> Artificial sequence

<220>
<223> NOTE: artificial peptide for polymer conjugates

<400> 40
Cys Trp Gly Gly Arg Gly Asp Ser Gly
1               5

<210> 41
<211> 9
<212> PRT
<213> Artificial sequence

<220>
<223> NOTE: artificial peptide for polymer conjugates

<400> 41
Cys Trp Gly Gly Arg Gly Asp Ser Tyr
1               5

<210> 42
<211> 9
<212> PRT
<213> Artificial sequence

<220>
<223> NOTE: artificial peptide for polymer conjugates

<400> 42
Cys Trp Gly Gly Arg Gly Asp Val Tyr
1               5

## Description

The present invention is concerned with materials and methods for mediating eukaryotic cell attachment to rigid surfaces. In particular, the invention is concerned with coatings for forming cell attachment surfaces, preferably for cell culture devices, microfluidic devices, biosensors or implants. The invention correspondingly provides polymer conjugates for such coatings, methods of polymer conjugate formation, methods of polymer conjugate application and uses of such polymer conjugates. The invention also provides surfaces and devices at least partly coated with such polymer conjugates.

### BACKGROUND OF THE INVENTION

For most non-degenerate eukaryotic cell lines of animal origin, cell cycle progression is inhibited unless attachment and spreading of such cells on a surface had been formed. Thus, cell adhesion is desired in particular in cell and tissue culture to achieve cell proliferation. Furthermore, cell adhesion is desirable for the generation of biological surface lining, in particular in biosensors where sustained presence of eukaryotic cells is required. Implants also benefit from a lining of non-degenerate cells to reduce inflammation reactions. Biocompatible materials for cell adhesion are thus generally well studied in the prior art. However, some materials like Matrigel(TM), fibronectin, gelatin, collagen or vitronectin can comprise mixtures of naturally produced substances. Such materials require extensive efforts for quality maintenance to prevent strong batchwise variation in product properties. Other materials are difficult to synthesize or purify to remove toxic or irritant substances.

It has been found that cell adhesion has effects beyond mere surface attachment and cell cycle progression. In particular, the mode of surface adhesion can influence the physiological state of the cells. For example, pluripotent stem cells undergo rapid differentiation dependent on the material offered for adhesion. Rapid differentiation is, however, undesirable for pluripotent stem cell expansion.

In view of the above described effects, the provision of materials to create suitable surfaces for cell adhesion without disruption of cell physiology, in particular without early induction of differentiation of pluripotent stem cells, has been the object of considerable research. However, such studies often did not provide suitable materials for stem cell expansion, mostly because they could not overcome the concupiscent inclination of pluripotent stem cells to differentiate. In particular it has been observed that even though numerous peptides, in particular those having at least one RGD motif, can be attached to surfaces to provide adhesion anchors for eukaryotic cells, none of them on their own prevented differentiation. This has led to the conclusion that while a ranking of different peptides with regards to cell attachment is in principle possible, such ranking only leads to qualitative results (Hersel et al., RGD-modified polymers: biomaterials for stimulated cell adhesion and beyond, Biomaterials 2003, 4385-4415). Complicating factors are, inter alia: A uniformly functionalised surface is obviously dissimilar to any natural cell environment, this alone is sufficient to influence cell development. Furthermore, even though the role of integrins in cell adhesion and differentiation is well studied, integrins in different states of activation can have different ligand binding activities. And cells interact with adjacent cells and the extracellular medium which results in differentiated, transient expression of integrins. Without any reliable guiding principles, finding a combination of materials suitable for cell adhesion and expansion has been akin to finding the proverbial needle in a haystack.

The invention is thus concerned with providing materials and methods to facilitate eukaryotic cell adhesion to surfaces. In particular, the materials should be easy to produce in consistently high quality, preferably measured by the number of stem cell culture passages before spontaneous differentiation occurs.

### SUMMARY OF THE INVENTION

Accordingly, the invention provides a cell attachment polymer conjugate, comprising a polymer and, conjugated thereto, a set of peptides, wherein
the peptides comprise, consist essentially of or consist of at least 3 peptides comprising two different integrin binding peptides and a positively charged peptide, preferably selected from the group consisting of
   a) a cyclic integrin binding peptide,
   b) a linear integrin binding peptide and
   c) a positively charged peptide,
and wherein the polymer comprises hydrophobic moieties and coupling moieties for conjugation of the peptides.

Correspondingly the invention also provides a cell attachment surface, comprising a substrate coated with the polymer conjugate of the present invention.

And the invention provides a cell attachment device comprising a cell attachment surface of the present invention.

Correspondingly the invention also provides a method of preparing a polymer conjugate according to the invention, comprising the steps of
i) providing a copolymer, wherein the amphiphilic polymer comprises a hydrophilic part selected from a polyethylene glycol, and a hydrophobic part, and
ii) conjugating peptides to said copolymer, wherein the peptides comprise, consist essentially of or consist of at least comprising two different integrin binding peptides and at least one positively charged peptide, preferably at least 3 peptides selected from the group consisting of a linear integrin binding peptide, a cyclic integrin binding peptide and a positively charged peptide.

The invention furthermore provides a method of preparing a cell attachment surface according or a cell attachment device according to the present invention, comprising the steps of
i) providing an amphiphilic copolymer, wherein the amphiphilic polymer comprises a hydrophilic part selected from a polyethylene glycol, and a hydrophobic part, and
ii) conjugating peptides to said copolymer, wherein the peptides comprise, consist essentially of or consist of at least 3 peptides comprising two different integrin binding peptides and a positively charged peptide, preferably selected from the group consisting of a linear integrin binding peptide, a cyclic integrin binding peptide and a positively charged peptide, and
iii) coating a substrate with the copolymer before, during or after step ii).

The invention also provides a use of a polymer conjugate of the present invention for the preparation of a cell attachment surface and/or cell attachment device for the cultivation of adrenal medulla cells, blood cells, bone marrow cells, cardiac muscle cells, endocrine cells, epidermal cells, epithelial cells, glandular cells, kidney cells, lung cells, muscle cells, neural cells, osteoblast cells, osteoclast cells, spleen cells, stem cells, thymus cells, degenerate cells and mixtures comprising such cells, preferably somatic or pluripotent stem cells. Furthermore, the invention provides a method of eukaryotic cell cultivation, comprising contacting eukaryotic cells with a polymer conjugate, a cell attachment surface or a cell attachment device, respectively, of the present invention.

And the invention provides the use of a polymer conjugate of the present invention for maintaining stem cells in a pluripotent state for at least 5 passages, more preferably 8-40 passages, more preferably 10-25 passages.

### BRIEF DESCRIPTION OF THE FIGURES

Figure 1 iPSCs on different polymer-peptide-conjugated substrates. iPSCs were grown on Ad1-polymer and Ad12-polymer and imaged over time compared to Matrigel. Scale bar 200 µm.
Figure 2 Adhesion and growth of iPSCs on differently coated surfaces. (A) IPSCs were cultured on different PPs and formed smaller colonies compared to iPSCs cultured on Matrigel. (B) Combinations of adhesive peptides with PP revealed bigger undifferentiated colonies (Ad12:PP3). Triple peptide mixtures promoted cell spreading even better than Ad12:PP3. Scale 200 µm.

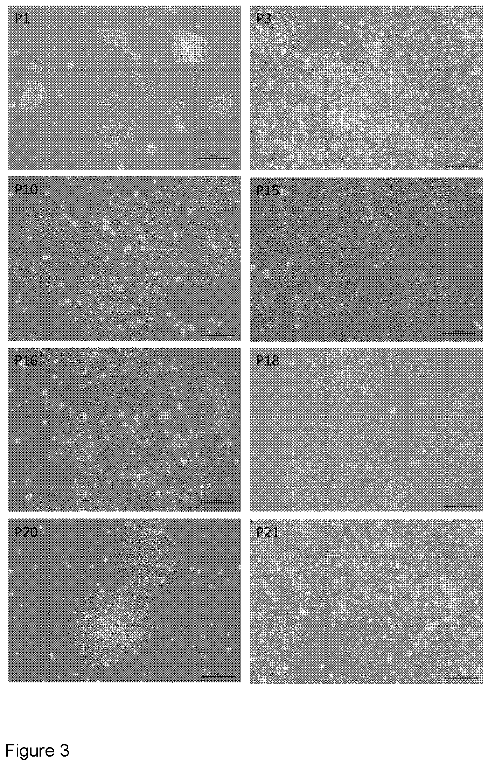

Figure 3: Microscopic images of iPSCs on polymer-peptide conjugate. Cells were seeded in P0 on polymer-peptide conjugate and split when 80% confluency was reached. iPSCs were cultured until passage 21 (21).
Figure 4: Analysis of pluripotency marker. A+B: Flow cytometry analysis for the pluripotency markers SSEA4 and TRA-1-60 of the cells grown on Matrigel or polymer-peptide. Comparable percentages of cells are double positive for these markers in both culture conditions. C: Flow cytometry analysis of OCT3/4+cells cultured on polymer-peptide conjugate in p21.

### DETAILED DESCRIPTION OF THE INVENTION

The technical teaching of the invention is expressed herein using the means of language, in particular by use of scientific and technical terms. However, the skilled person understands that the means of language, detailed and precise as they may be, can only approximate the full content of the technical teaching, if only because there are multiple ways of expressing a teaching, each necessarily failing to completely express all conceptual connections, as each expression necessarily must come to an end. With this in mind the skilled person understands that the subject matter of the invention is the sum of the individual technical concepts signified herein or expressed, necessarily in a pars-pro-toto way, by the innate constrains of a written description. In particular, the skilled person will understand that the signification of individual technical concepts is done herein as an abbreviation of spelling out each possible combination of concepts as far as technically sensible, such that for example the disclosure of three concepts or embodiments A, B and C are a shorthand notation of the concepts A+B, A+C, B+C, A+B+C. In particular, fallback positions for features are described herein in terms of lists of converging alternatives or instantiations. Unless stated otherwise, the invention described herein comprises any combination of such alternatives. The choice of more or less preferred elements from such lists is part of the invention and is due to the skilled person's preference for a minimum degree of realization of the advantage or advantages conveyed by the respective features. Such multiple combined instantiations represent the adequately preferred form(s) of the invention. Any recited single or multiple feature or aspect in any one claim, example, embodiment or other teaching described herein can be combined or permuted with any other recited feature or aspect in any other claim, example, embodiment or teaching described herein.

As used herein, terms in the singular and the singular forms like "a", "an" and "the" include plural referents unless the content clearly dictates otherwise. Thus, for example, use of the term "a nucleic acid" optionally includes, as a practical matter, many copies of that nucleic acid molecule; similarly, the term "probe" optionally (and typically) encompasses many similar or identical probe molecules. Also as used herein, the word "comprising" or variations such as "comprises" or "comprising" will be understood to imply the inclusion of a stated element, integer or step, or group of elements, integers or steps, but not the exclusion of any other element, integer or step, or group of elements, integers or steps. The same applies to the term "preferably", which is only implying a preference without intention of limitation of the scope of the invention or of the scope of broader claims. In particular, the term "preferably" is not meant as a disparagement of less preferred variants or alternatives.

As used herein, the term "and/or" refers to and encompasses any and all possible combinations of one or more of the associated listed items, as well as the lack of combinations when interpreted in the alternative ("or"). The term "comprising" also encompasses the term "consisting of".

The term "about", when used in reference to a measurable value, for example an amount of mass, dose, time, temperature, sequence identity and the like, refers to a variation of ± 0.1%, 0.25%, 0.5%, 0.75%, 1%, 2%, 3%, 4%, 5%, 6%, 7%, 8%, 9%, 10%, 15% or even 20% of the specified value as well as the specified value. Thus, if a given composition is described as comprising "about 50% X," it is to be understood that, in some embodiments, the composition comprises 50% X whilst in other embodiments it may comprise anywhere from 40% to 60% X (i.e., 50% ± 10%).

Stem cells according to the present invention are somatic or pluripotent stem cells excluding stem cells obtained from human embryos other than umbilical cord derived stem cells. Preferably stem cells are induced pluripotent stem cells.

The invention provides a cell attachment polymer conjugate. The polymer conjugate comprises an amphiphilic polymer and, conjugated thereto, a set of peptides. It is a particular advantage of the present invention that the peptides can be chemically synthesized according to methods known in the art and do not require isolation and/or fractionation from natural sources.

The peptides comprise, consist essentially of or consist of at least two different integrin binding peptides and a positively charged peptide. Preferably at least 1 peptide is selected from each of the groups consisting of linear integrin binding peptides, cyclic integrin binding peptides and positively charged peptides. The peptides expediently allow for the coupling of the polymer, preferably by providing a coupling moiety. The polymer, in turn, comprises hydrophobic moieties and, for conjugation of the peptides, coupling moieties. Without being bound to any particular theory, it is currently thought that positively charged peptides are particularly useful because they counteract negative charges in cadherin, a ubiquitous cell surface protein.

It has now surprisingly been found that the combination of peptides as proposed by the present invention, when conjugated to the polymer basis, advantageously allows for a stable maintenance of undifferentiated pluripotent stem cells even in the absence of differentiation inhibitors or feeder layers, e.g. mouse fibroblasts. The invention thus advantageously provides a purely synthetic material for coating of surfaces to facilitate stable eukaryotic cell adhesion. It is a further advantage of the present invention that the polymer conjugate is stable, after conjugation of the peptides, at common laboratory storage conditions (4°C in the dark) for at least 3 months, preferably in the dark at 4°C for 6 months. Thus, the invention allows to store the polymer conjugate, or, as described below in greater detail, a kit comprising the polymer and the set of peptides, and to prepare cell adhesion surfaces freshly at the desired time.

The following tables list preferred peptides. Small non-italicised letters indicate D-amino acids, the designation "c(XXX)" indicates a cyclic peptide of sequence XXX. Many of the peptides preferably selected according to the present invention are individually known in the prior art:

| Name | SEQ ID NO. | Sequence | Reference |
|---|---|---|---|
| Ad1 | 1 | CWGGRGDSP | Caiazzo, M., et al., Defined three-dimensional microenvironments boost induction of pluripotency. Nat Mater, 2016. 15(3): p. 344-52. |
| Ad1c | 2 | c(RGDyC) | Nguyen, E.H., et al., Versatile synthetic alternatives to Matrigel for vascular toxicity screening and stem cell expansion. Nature biomedical engineering, 2017. 1: p. 0096. |
| Ad12 | 3 | GCWGGPQVTRGDVFTMP | Dolley-Sonneville, P.J., L.E. Romeo, and Z.K. Melkoumian, Synthetic Surface for Expansion of Human Mesenchymal Stem Cells in Xeno-Free, Chemically Defined Culture Conditions. PLOSONE, 2013. 8(8): p. e70263. |
| Ad21 | 4 | GCWGGQPPRARITGYII | Hozumi, K., etl al., Mixed Fibronectin-Derived Peptides Conjugated to a Chitosan Matrix Effectively Promotes Biological Activities through Integrins, alpha4beta1, alpha5beta1, alphavbeta3, and Syndecan, Bioresearch Open Access, 5(1): p. 356 |
| Ad22 | 5 | GCWWGGNGEPRGDTYRAY | Senta,H., et al., Combination of synthetic peptides derived from bone morphogenetic proteins and biomaterials for medical applications. The Canadian Journal of Chemical Engineering, 2011. 89:p 227 |
| PP3 | 6 | GCWGRKKGRKKGRKKGRKK | Tsuchiya, K., et al., Effects of cell adhesion molecules on adhesion of chondrocytes, ligament cells and mesenchymal stem cells. Materials Science and Engineering: C, 2001. 17(1): p. 79-82. |
| PP4 | 7 | GCWGKKRGKKRGKKRGKKR | Tsuchiya, K., et al., Effects of cell adhesion molecules on adhesion of chondrocytes, ligament cells and mesenchymal stem cells. Materials Science and Engineering: C, 2001. 17(1): p. 79-82. |
| PP2 | 8 | GCWGRRRARGKKKAK | Tsuchiya, K., et al., Effects of cell adhesion molecules on adhesion of chondrocytes, ligament cells and mesenchymal stem cells. Materials Science and Engineering: C, 2001. 17(1): p. 79-82. |
| RGDS | 9 | RGDS | |
| RGDT | 10 | RGDT | |
| RGDV | 11 | RGDV | |
| GRGD | 12 | GRGD | |
| GRGDG | 13 | GRGDG | |
| GRGDF | 14 | GRGDF | |
| GRGDS | 15 | GRGDS | |
| GRGDY | 16 | GRGDY | |
| YRGDG | 17 | YRGDG | |
| YRGDS | 18 | YRGDS | |
| YGRGD | 19 | YGRGD | |
| RGDSG | 20 | RGDSG | |
| RGDSP | 21 | RGDSP | |
| RGDSY | 22 | RGDSY | |
| RGDVY | 23 | RGDVY | |
| GRGDSP | 24 | GRGDSP | |
| GRGDSG | 25 | GRGDSG | |
| GRGDSY | 26 | GRGDSY | |
| GRGDVY | 27 | GRGDVY | |
| GRGDSPK | 28 | GRGDSPK | |
| CGRGDSPK | 29 | CGRGDSPK | |
| CGRGDSY | 30 | CGRGDSY | |
| RGDfK | 31 | RGDfK | |
| YAVTGRGDS | 32 | YAVTGRGDS | |
| CGGNGEPRGDYRAY | 33 | CGGNGEPRGDYRAY | |
| GCGYGRGDSPG | 34 | GCGYGRGDSPG | |
| RGDSPASSKP | 35 | RGDSPASSKP | |
| GRGDSPASSKG | 36 | GRGDSPASSKG | |
| CWGGRGDS | 37 | CWGGRGDS | |
| CWGGRGDT | 38 | CWGGRGDT | |
| CWGGRGDV | 39 | CWGGRGDV | |
| CWGGRGDSG | 40 | CWGGRGDSG | |
| CWGGRGDSY | 41 | CWGGRGDSY | |
| CWGGRGDVY | 42 | CWGGRGDVY | |

| Name | Reference |
|---|---|
| c(phg-isoDG RX) | Bochen, A. et al. Biselectivity of isoDGR peptides for fibronectin binding integrin subtypes α 5β 1 and α vβ 6: conformational control through flanking amino acids. J. Med. Chem. 56, 1509-1519 (2013). |
| c(RGDfC) | Prante, O. et al. 3,4,6-Tri-O-acetyl-2-deoxy-2-[18F]fluoroglucopyranosyl phenylthiosulfonate: a thiol-reactive agent for the chemoselective 18F-glycosylation of peptides. Bioconjugat. Chem. 18, 254 (2007). |
| c(RGDfK) | Kantlehner, M. et al. Surface Coating with Cyclic RGD Peptides Stimulates Osteoblast Adhesion and Proliferation as well as Bone Formation. ChemBioChem 1, 107-114 (2000). |
| c(RGDfV) | Aumailley, M. et al. Arg-Gly-Asp constrained within cyclic pentapeptides. Strong and selective inhibitors of cell adhesion to vitronectin and laminin fragment P1. FEBS Lett. 291, 50-54 (1991). |
| c(RGDyK) | Brooks, P. C. et al. Integrin alpha v beta 3 antagonists promote tumor regression by inducing apoptosis of angiogenic blood vessels. Cell 79, 1157-1164 (1994). |
| GRGDNP | Mogford, J. E., Davis, G. E. & Meininger, G. A. RGDN Peptide Interaction with Endothelial a5b1 Integrin Causes Sustained Endothelin-dependent Vasoconstriction of Rat Skeletal Muscle Arterioles, J. Clin. Invest. 100, 1647-1653 (1997). |
| GRGDTP | Dedhar, S., Ruoslahti, E. & Pierschbacher, M. D. A Cell Surface Receptor Complex for Collagen Type I Recognizes the Arg-Gly-Asp Sequence. J. Cell. Biol. 104, 585-593 (1987). |
| RTDLDSLRT | Kraft, S. et al. Definition of an unexpected ligand recognition motif for alphav beta6 integrin. J. Biol. Chem. 274, 1979-1985 (1999). |
| (Ga)NOPO*-c(RGDfK) | Šimeček, J. et al. Benefits of NOPO As Chelator in Gallium-68 Peptides, Exemplified by Preclinical Characterization of 68Ga-NOPO-c(RGDfK). Mol. Pharm. 11, 1687- 1695 (2014). |
| 44b | Heckmann, D. et al. Rational Design of Highly Active and Selective Ligands for the α 5β 1 Integrin Receptor. ChemBioChem 9, 1397-1407 (2008). |
| A20FMDV2 | DiCara, D. et al. Structure-Function Analysis of Arg-Gly-Asp Helix Motifs in α vβ 6 Integrin Ligands. J. Biol. Chem. 282, 9657-9665 (2007). |
| ATN161 | Stoeltzing, O. et al. Inhibition of integrin alpha5beta1 function with a small peptide (ATN-161) plus continuous 5-FU infusion reduces colorectal liver metastases and improves survival in mice. Int. J. Cancer 104, 496-503 (2003). |
| c(FRGDLAFp(NMe)K) | Maltsev, O. V. et al. Stable Peptides Instead of Stapled Peptides: Highly Potent α vβ 6-Selective Integrin Ligands, Angew. Chem. 128, 1559-1563 (2016). Angew. Chem. Int. Ed. 55, 1535-1538 (2016). |
| Cilengitide | Dechantsreiter, M. A. et al. N-Methylated cyclic RGD peptides as highly active and selective alpha(V)beta(3) integrin antagonists. J. Med. Chem. 42, 3033-3040 (1999). |
| Echistatin | Gan, Z. R., Gould, R. J., Jacobs, J. W., Friedman, P. A. & Polokoff, M. A. Echistatin - A potent platelet aggregation inhibitor from the venom of the viper, Echis Carinatus. J. Biol. Chem. 263, 19827-19832 (1988). |
| Eptifibatide | Scarborough R. M., Naughton M. A., Teng W. et al. Design of potent and specific integrin antagonists. Peptide antagonists with high specificity for glycoprotein IIb-IIIa. J Biol Chem 268, 1066-73 (1993). |
| F-Galacto-c(RGDfK) | Haubner, R. et al. Noninvasive Visualization of the Activated α vβ 3 Integrin in Cancer Patients by Positron Emission Tomography and [18F]Galacto-RGD. PLoS Medicine 2, 244-252 (2005). |
| Flucilatide | Kenny, L. M. et al. Phase I Trial of the Positron-Emitting Arg-Gly-Asp (RGD) Peptide Radioligand 18F-AH111585 in Breast Cancer Patients. J. Nucl. Med. 49, 879-886 (2008). |
| GR144053 | Eldred, C. D. et al. Orally active non-peptide fibrinogen receptor (GpIIb/IIIa) antagonists: identification of 4-[4-[4-(aminoiminomethyl)phenyl]-1-piperazinyl]-1-piperidineacetic acid as a long-acting, broad spectrum antithrombotic agent. J. Med. Chem. 37, 3882-3885 (1994). |
| JSM6427 | Stragies, R. et al. Design and synthesis of a new class of selective integrin alpha5beta1 antagonists. J. Med. Chem. 50, 3786-3794 (2007). |
| Mo/11* | Goodman, S. L., Hölzemann, G., Sulyok, G. A. & Kessler, H. Nanomolar small molecule inhibitors for alphav(beta)6, alphav(beta)5, and alphav(beta)3 integrins. J. Med. Chem. 45, 1045-1051 (2002). |
| NC100717 | Indrevoll, A. et al. NC-100717: A versatile RGD peptide scaffold for angiogenesis imaging. Bioorg. Med. Chem. Lett. 16, 6190-6193 (2006). |
| RGD-4C | Smolarczyk, R. et al. Antitumor effect of RGD-4C-GG-D(KLAKLAK)2 peptide in mouse B16(F10) melanoma model. Acta Biochim. Pol. 53, 801-805 (2006). |
| RGD10 | Hölig, P. et al. Novel RGD lipopeptides for the targeting of liposomes to integrin-expressing endothelial and melanoma cells. Protein Eng. Des. Sel. 17, 433-441 (2004). |
| sn243 | Neubauer, S. et al. Pharmacophoric Modifications Lead to Superpotent α vβ 3 Integrin Ligands with Suppressed α 5β 1 Activity; J. Med. Chem. 57, 3410-3417 (2014). |
| Tirofiban | Hartman, G. D. et al. Non-peptide fibrinogen receptor antagonists. Discovery and design of exosite inhibitors. J. Med. Chem. 35, 4640-4642 (1992). |

The peptides can consist of the aforementioned peptides. Preferably the peptides comprise at least 1, more preferably comprising 3 peptides from the aforementioned tables. Among those peptides the sequences selected from SEQ ID NO. 1-41 and Cilengitide, Echistatin and Eptifibatide are particularly preferred. More preferred is a set of peptides comprising three peptides selected from SEQ ID NO. 1-8 (Ad1, Ad1c, Ad12, Ad21, Ad22, PP3, PP2, PP4). Even more preferably the set comprises Ad1c, one or two peptides of the group consisting of Ad1, Ad12, Ad21 and Ad22 and at least one peptide of the group consisting of PP2, PP3 and PP4. Even more preferably the set comprises Ad1c, PP3 and one peptide selected from Ad1, Ad12, Ad21 and Ad22.

Each peptide of the aforementioned tables can also be a variant peptide differing by up to 4 amino acid positions from the respective sequence, more preferably by at most 3 amino acid positions, more preferably by at most 2 amino acid positions, even more preferably by at most 1 amino acid position. However, each variant peptide must still allow for coupling to at least one type of coupling moieties of the polymer. The differences are selected from additions at the end of a sequence, amino acid insertions, amino acid substitutions and amino acid deletions. In case of substitutions, the replaced amino acids are preferably chosen from the following table:

| Original | Replacement, higher numbers denote higher degree of preference |
|---|---|
| A | S (1), G (0), T (0) |
| R | K (2), Q (1), H (0) |
| N | D (1), Q (0), H (0), K (0), S (0), T (0) |
| D | N (1), E (1) |
| C | |
| Q | E (2), H (1), K (1), R (1), N (0), M (0) |
| E | Q (2), D (1), K (0) |
| G | A (0) |
| H | Q (1), Y (1), R (0), N (0) |
| I | V (3), L (1), M (1) |
| L | M (2), I (1), F (0), V (0) |
| K | R (2), Q (1), N (0), E (0) |
| M | L (2), I (1), Q (0), V (0) |
| F | Y (3), L (0), W (0) |
| P | |
| S | A (1), T (1), N (0) |
| T | S (1), A (0), N (0) |
| W | Y (2), F (0) |
| Y | F (3), W (2), H (1) |
| V | I (3), L (0), M (0) |

Preferably the peptides comprise, consist essentially of or consist of peptides selected from Ad1c, Ad12, PP3 and peptides differing from Ad1c, Ad12 or PP3, respectively, by at most 1 amino acid difference. As is shown in the examples, this combination of peptides on a suitable polymer allows for the preparation of a particularly advantageous cell attachment surface coating. In particular, surfaces coated with such a polymer conjugate not only allow stem cells to stably adhere to the surface in a phenotypically healthy colony morphology, the stem cells also remain in an undifferentiated stage over repeated passages (more than 10) from one such surface to another. This is all the more remarkable as no differentiation inhibitor substance nor feeder layers had to be applied to prevent or delay differentiation. For the avoidance of doubt: The invention is not restricted to operate in an environment free of differentiation inhibitors. However, substantial benefits of the invention can already be achieved in the absence of differentiation inhibitors.

It is particularly preferred that the set of peptides comprises
a) at least 1 cyclic integrin-binding peptide, preferably 1-5 cyclic integrin-binding peptides, wherein a particularly preferred cyclic integrin-binding peptide is Ad1c or peptides differing therefrom by at most 1 amino acid difference,
b) at least 1 linear integrin-binding peptide, preferably 1-5 linear integrin-binding peptides, wherein a particularly preferred linear integrin-binding peptide is Ad12 or peptides differing therefrom by at most 1 amino acid difference, and
c) at least 1 positively charged peptide, preferably 1-5 positively charged peptides, wherein a particularly preferred positively charged peptide is PP3 or peptides differing therefrom by at most 1 amino acid difference,
wherein the molar ratio of peptides according to a):b):c) is (0.5-4):1:(0.01-1), more preferably (0.7-3):1:(0.08-0.8), more preferably (1.5-2.5):1:(0.1-0.4).

As is shown in the examples below, providing the aforementioned peptides in said ratios particularly increases the number of passages of stem cell cultures without differentiation.

Further preferably, the molar ratio of the total of Ad1c, Ad12 and PP3 to the total of other peptides is 2:1, more preferably 3:1, more preferably 5:1, more preferably 10:1, and most preferably the set consists of Ad1c, Ad12 and PP3.

It is particularly preferred that the total concentration of peptides of the set of peptides and of the polymer is adjusted such that, upon coating of a surface with the polymer conjugate of the invention, a density of at least 12 pmol of the peptides of the set of peptides per square cm is achieved, more preferably a density of 12-200 pmol/cm², more preferably a density of about 15-180 pmol/cm², more preferably a density of about 20-100 pmol/cm², more preferably a density of about 30-100 pmol/cm². With such densities a particularly efficient expansion of pluripotent stem cells without differentiation is possible.

The set of peptides is provided in a concentration sufficient to achieve conjugation of at least about 50% of conjugation sites of the polymer, more preferably about 60-100% of the conjugation sites, more preferably about 70-100% of the conjugation sites, more preferably 75-90% of the conjugation sites, more preferably about 75-100% of the conjugation sites, more preferably 80-95% of the conjugation sites, more preferably about 80-100% of the conjugation sites, more preferably about 90-100% of the conjugation sites, more preferably about 100% of the conjugation sites. It is a particular advantage of the present invention that the polymer conjugate may also comprise, conjugated to the polymer, further substances other than the set of peptides. Thus, the polymer conjugate of the present invention is advantageously versatile and allows to provide more functions in addition to e.g. adhesion and/or maintenance and expansion of undifferentiated stem cells. Further substances preferably conjugated to the polymer are selected from structural proteins (for example collagen and elastin), proteoglycans, polysaccharides and glycoproteins (for example glycosaminoglycans, in particular hyaluronic acid).

The **polymer conjugate** may have an architecture of a head-to-tail conjugate, a comb-shaped structure, or a dendritic structure, wherein the head-to-tail or comb-shaped structure conjugate are preferred. Typically, in the head-to-tail conjugate a peptide is conjugated to one or both ends of a linear polymer. Typically, in a comb-shaped structure at least one peptide is conjugated along the backbone of a linear polymer. Typically, in a dendritic structure at least one peptide is conjugated to the end of a dendritic polymer.

The peptide is **conjugated** to the amphiphilic polymer, which usually means it is covalently linked to the amphiphilic polymer. The conjugation can be made with or without a linker, preferably with a linker. Typically, the conjugation uses an activating reagent. In a preferred form the peptide is conjugated to the amphiphilic polymer by using an activating reagent and a linker. The conjugation of a peptide to a polymer and the preparation of the polymer conjugate is known to an expert, e.g from the following reviews: Canalle et al., Chem. Soc. Rev. 2010, 39, 329-353; Chen et al., Prog. Polym. Sci. 2020, 105, 101241.

Suitable classes of **activating reagents** are amine-reactive activating reagents, carboxylic acid-reactive activating reagents and sulfhydryl-reactive activating reagents. The activating reagents can react with specific functional groups at the peptide (such as sulfhydryl groups) and the amphiphilic polymer (such as carboxylic acid or amine groups). Further, chemoselective azide-alkyne cycloaddition (also called click technology) can be used for conjugation, or a copper-free click chemistry (also known as strain-promoted alkyne azide cycloaddition (SPAAC).

Suitable **amine-reactive activating reagents** are N-hydroxysuccinimide esters (NHS esters), Sulfo-NHS esters (e.g. N-hydroxysulfosuccinimide) and imidoesters.

Suitable NHS esters can be reactive groups formed by EDC (1-ethyl-3-(3-dimethylaminopropyl)carbodiiimide) activation of carboxylate groups. Sulfo-NHS esters are typically like NHS esters except that they contain a sulfonate (-SO₃) group on the N-hydroxysuccinimide ring. Suitable NHS ester are disuccinimidyl glutarate, disuccinimidyl suberate, bis(sulfosuccinimidyl)suberate, PEGylated bis(sulfosuccinimidyl)suberate, dithiobis(succinimidyl propionate), 3,3'-dithiobis(sulfo¬succinimidyl propionate), disuccinimidyl tartrate, bis(2-(succinimidooxycarbonyloxy) ethyl)sulfone), ethylene glycol bis(succinimidyl succinate), ethylene glycol bis(sulfosuccinimidyl succinate), tris-(succinimidyl) aminotriacetate).

Suitable imidoesters are dimethyl adipimidate, dimethyl pimelimidate, dimethyl suberimidate, 1,5-difluoro-2,4-dinitrobenzene.

Suitable **carboxylic acid-reactive activating reagents** are carbodiimides, aminium reagents and phosphonium reagents.

Suitable carbodiimides are 1-ethyl-3-(3-dimethylaminopropyl carbodiimide (EDC), diisopropylcarbodiimide (DIC), N-cyclohexyl-N'-(2-morpholinoethyl)carbodiimide-methyl-p-toluenesulfonate (CMC) and dicyclohexyl carbodiimide (DCC). N-hydroxysuccinimide (NHS) or its water-soluble analog (sulfo-NHS) is often included in EDC coupling protocols.

Suitable aminium reagents (formerly known as uronium reagents) and phosphonium reagents are
- benzotriazole-1-yl-oxy-tris-(dimethylamino)-phosphonium hexafluorophosphate (BOP), benzotriazole-1-yl-oxy-tris-pyrrolidino-phosphonium hexafluorophosphate (PyBOP),
- Brom-tripyrrolidinophosphonium-hexafluorophosphate (PyBrOP),
- [Ethyl-cyano-(hydroxyimino)-acetato-O2]-tri-1-pyrrolidinylphosphonium-hexafluorophosphat (PyOxim),
- (7-azabenzotriazol-1-yloxy)trispyrrolidinophosphonium hexafluorophosphate (PyAOP), TBTU,
- 2-(1H-benzotriazole-1-yl)-1,1,3,3-tetramethyluronium hexafluorophosphate (HBTU),
- O-(7-azabenzotriazole-1-yl)-N,N,N',N'-tetramethyluronium tetrafluoroborate (TATU),
- HATU (CAS 148893-10-1),
- O-(1H-6-chlorobenzotriazole-1-yl)-1,1,3,3-tetramethyluronium hexafluorophosphate (HCTU), HDMC (CAS 1082951-62-9),
- 1-[(1-(cyano-2-ethoxy-2-oxoethylideneaminooxy) dimethylaminomorpholino)] uronium hexafluorophosphate (COMU),
- fluoro-N,N,N',N'-tetramethylformamidinium hexafluorophosphate (TFFH),
- O-(5-norbornen-2,3-dicarboximido)-N,N,N',N'-tetramethyluronium-tetrafluoroborat (TNTU), O-(N-Succinimidyl)-1,1,3,3-tetramethyluronium Tetrafluoroborate (TSTU),
- 4-(4,6-dimethoxy-1,3,5-triazin-2-yl)-4-methyl-morpholinium chloride (DMTMM).

The amimium reagents and phosphonium reagents are usually used in combination with a base, preferably a tertiary base, for example N,N-diisopropylethylamine (DIPEA) or N-methylmorpholine (NMM). The carboxylic acid group is usually activated beforehand with NHS or sulfo-NHS, and/or with additives, such as 1-hydroxy-1H-benzotriazol (HOBt), 1-hydroxy-7-azabenzotriazol (HOAt) or ethyl cyano(hydroxyimino)acetate (Oxyma).

Preferred activating reagents are water soluble at the typical process conditions. Suitable water soluble activating reagents are EDC, DMTMM, CMC, TNTU, TSTU.

Suitable **linkers** are maleimides, haloacetyls or pyridiyl disulfides, wherein maleimides are preferred.

Suitable maleimides are bismaleimidoethane, 1,4-bismaleimidobutane, bismaleimidohexane, 1,8-bismaleimidodiethyleneglycol, 1,11-bismaleimidotriethyleneglycol, dithiobismaleimidoethane, tris(2-maleimidoethyl)amine, 1-(2-aminoethyl)maleimide, N-(3-aminopropyl)maleimide, maleimide-poly(ethylene glycol)-amine, 1-(4-aminophenyl)-1H-pyrrole-2,5-dione, (N-(β-maleimidopropionic acid) hydrazide, (N-ε-maleimidocaproic acid hydrazide), (4-(4-N-maleimidophenyl) butyric acid hydrazide), N-κ-maleimidoundecanoic acid hydrazide.

Preferred maleimides are 1-(2-aminoethyl)maleimide, N-(3-aminopropyl)maleimide, maleimide-poly(ethylene glycol)-amine, maleimide-poly(ethylene glycol)-amine, 1-(4-aminophenyl)-1H-pyrrole-2,5-dione, (N-((3-maleimidopropionic acid) hydrazide, (N-ε-maleimidocaproic acid hydrazide), (4-(4-N-maleimidophenyl) butyric acid hydrazide), N-κ-maleimidoundecanoic acid hydrazide.

Suitable haloacetyls are succinimidyl iodoacetate, succinimidyl 3-(bromoacetamido) Propionate, succinimidyl (4-iodoacetyl) aminobenzoate, sulfosuccinimidyl (4-iodoacetyl)aminobenzoate.

Suitable pyridyl disulfides are 3-(2-pyridyldithio)propionyl hydrazide.

In a preferred form the peptide is conjugated to the amphiphilic polymer by using an activating reagent selected from carboxylic acid-reactive activating reagents (preferably carbodiimides and aminium reagents), and a linker selected from maleimides.

The **amphiphilic polymer** can comprise a hydrophilic part and a hydrophobic part. The architecture of the parts is usually a block polymer (e.g. a di-block or tri-block polymer, such as A-B or B-A-B, or A-B-A) or a comb-like polymer (e.g. with hydrophobic part in the backbone and hydrophilic part in the side-chains).

The hydrophilic part comprises usually a polyethylene glycol. The polyethylene glycol can have a C1-6 alkyl ether on one end, such as a methyl ether. The molecular weight of the hydrophilic part (e.g. the polyethylene glycol) can be from 100 to 50.000 g/mol, preferably from 200 to 15.000, and in particular from 300 to 5.000 g/mol.

The hydrophobic part comprises usually a polyalkylene glycol based on C3-C12 alkylene oxides (preferably C3-C5 alkylene oxide, in particular propylene oxide), or a hydrophobic polymer based on a hydrophobic monomer. The molar amount of the hydrophobic monomer in the hydrophobic polymer can be adjusted in broad ranges, such as 10-90 mol%, preferably 30-70 mol%.

Suitable hydrophobic monomers are for example
- aromatic monomers, such as styrene, phenyl acrylate, benzyl acrylate, phenylethyl acrylate and phenoxyethyl acrylate;
- C1-C18 alkyl (meth)acrylates (preferably C1-4 alkyl (meth)acrylates), such as methyl (meth)acrylate, ethyl (meth)acrylate, n-butyl (meth)acrylate, tert-butyl (meth)acrylate, propyl (meth)acrylate, pentyl (meth)acrylate, hexyl (meth)acrylate, cyclohexyl (meth)acrylate, heptyl (meth)acrylate, octyl (meth)acrylate, nonyl (meth)acrylate, decyl (meth)acrylate, dodecyl (meth)acrylate;
- C4-18 alkenes, such as isobutylene, diisobutylene, 1-decene, 1-dodecene, C18 alpha olefine;
- methyl vinyl ether; and
- vinylpyrrolidone.

Preferred hydrophobic monomers are aromatic monomers (such as styrene) and C1-C18 alkyl (meth)acrylates (such as C1-4 alkyl (meth)acrylates), wherein aromatic monomers are most preferred.

The hydrophobic part is preferably a polypropylene glycol, or a hydrophobic polymer based on an aromatic monomer, such as styrene.

The amphiphilic polymer usually comprises at least one linker-reactive group, which may be a functionalized monomer or a terminal group. Examples of terminal groups are hydroxy or amine groups. Examples for functionalized monomers are monomers with acids, amine, or sulfhydryl functional groups, such as maleic acid, (meth)acrylates (e.g. methacrylic acid) or (meth)acrylamides with functional groups. The molar amount of the linker-reactive groups in the amphiphilic polymer can be adjusted in broad ranges, such as 0.1-70 mol%, preferably 1-60 mol%.

In one form the hydrophobic polymer contains further comonomers, such as the functionalized monomer suitable for reacting with the linker. The molar amount of the further comonomer in the hydrophobic polymer can be adjusted in broad ranges, such as 1-70 mol%, preferably 5-60 mol%.

In a preferred form the amphiphilic polymer comprises a hydrophilic part selected from a polyethylene glycol, and a hydrophobic part.

In a preferred form the amphiphilic polymer comprises
a hydrophilic part selected from a polyethylene glycol, and
a hydrophobic part selected from a polypropylene glycol, or a hydrophobic polymer based on an aromatic monomer (such as styrene) or C1-C18 alkyl (meth)acrylates (such as C1-4 alkyl (meth)acrylates).

In another preferred form the amphiphilic polymer comprises
a hydrophilic part selected from a polyethylene glycol which may optionally have a methyl ether at one end, and
a hydrophobic part selected from a hydrophobic polymer-based styrene and a further comonomer selected from maleic acid.

In another preferred form the amphiphilic polymer comprises a hydrophilic part selected from a polyethylene glycol which has a methyl ether at one end, and
a hydrophobic part selected from a hydrophobic polymer based styrene and a further comonomer selected from maleic acid, where the polyethylene glycol is attached to parts of the maleic acid.

In a preferred form the amphiphilic polymer comprises
a hydrophilic part selected from a polyethylene glycol, and
a hydrophobic part selected from a hydrophobic polymer based on C1-C18 alkyl (meth)acrylates (such as C1-4 alkyl (meth)acrylates) and optionally a further comonomer selected from (meth)acrylic acid.

In a preferred form the amphiphilic polymer comprises
a hydrophilic part selected from a polyethylene glycol, and
a hydrophobic part selected from a hydrophobic polymer based on C1-4 alkyl (meth)acrylates and a further comonomer selected from (meth)acrylic acid, where the polyethylene glycol is attached to parts of the (meth)acrylic acid.

In another preferred form the amphiphilic polymer comprises
a hydrophilic part selected from a polyethylene glycol, and
a hydrophobic part selected from a polypropylene glycol,
where the amphiphilic polymer can be a diblock or triblock polymer.

The amphiphilic polymer may have a molecular weight of 5000 to 500.000 g/mol, preferably 10.000 to 100.000 g/mol. The molecular weight can be determined by gel permeation chromatography.

The invention also provides a cell attachment surface, comprising a substrate coated with the polymer conjugate of the present invention. As shown herein, such surfaces advantageously allow to prepare animal eukaryotic cell cultures adherent to the coated substrate, wherein the cultures are phenotypically healthy. It is a particular advantage that the coated surfaces can be prepared very easily, as described below, from fully synthetic, toxin and animal-free compounds. In particular, cell attachment surfaces according to the present invention advantageously help maintain stem cells in an undifferentiated form even in the absence of feeder cell layers or added differentiation inhibitors.

In general, the whole surface or parts of the surface comprises an adsorbed layer of the polymer conjugate. Preferably, at least the part of the surface which usually comes into contact with a biological material (e.g. cells) under normal operating conditions comprises the adsorbed layer of the polymer conjugate. The layer of the polymer conjugate is adsorbed to the surface, which usually means that there are no covalent chemical bonds between the polymer conjugate and the surface. The term "adsorbed" usually refers to physisorption, and usually not to chemisorption.

Preferably, the surface is free of other adsorbed polymer conjugate beside the polymer conjugate. Preferably, the surface is free of other layers (e.g. adsorbed, or covalently bound layers) beside the adsorbed layer of the polymer conjugate.

The adsorbed layer may comprise at least one polymer conjugate, such as one, two or three different polymer conjugates. Preferably, the adsorbed layer consists of the polymer conjugate. In one form the adsorbed layer is free of other polymer conjugates beside the polymer conjugate. In another form the adsorbed layer is free of biological compounds, pharmaceuticals or biologically active compounds.

The cell attachment surface can be made of any biocompatible material, e.g. material on which cells can be grown. For example, the surface is at least partly made of glass, quartz, silicon, metals, metal oxides or organic polymers.

Suitable glass can be fused quartz glass (also called fuse silica), soda-lime glass, borosilicate glass, lead glass, aluminosilicate glass, glass-ceramics and fiberglass. Preferred material is fused quartz glass and soda-lime glass.

In another form, the surface is at least partly made of organic polymers, such as polycarbonate, polystyrene, hydrophilized polystyrene, polyamide, poly(methyl methacrylate), polyesters, polysulfones (like polyethersulfones), polyvinylchloride, polyvinylidene chloride, fluorinated or partially fluorinated polyolefins (like fluorinated polyethylene or polypropylene), polyolefines [such as polyethylene (like low density polyethylene, ultralow density polyethylene, linear low density polyethylene, high density polyethylene, high molecular weight polyethylene, ultrahigh molecular weight polyethylene), polypropylene (like oriented polypropylene, biaxially oriented polypropylene), cyclic olefin polymers (COP, like polynorbornene) or cyclic olefin copolymers (COC, like copolymers of ethylene and norbornene)].

In another form, the surface is at least partly made of polystyrene or hydrophilized polystyrene.

The term "surfaces is at least partly made of a material" usually means that at least 50 %, preferably at least 80%, and in particular at least 95% of the surface is made of the material. The surface usually refers to that part of the surface of the device which in general comes into contact with biological material (e.g. cells) under normal operating conditions. In a form the at least 50 %, preferably at least 80%, and in particular at least 95% of the surface is made of glass.

Correspondingly the invention also provides a cell attachment device comprising a cell attachment surface according the present invention. A cell attachment device allows to implement the advantages conferred by the polymer conjugate of the present invention, in particular in eukaryotic cell culture settings.

The cell attachment device of the present invention is thus preferably a cell culture device for cultivating eukaryotic cells. Preferably the cell culture device is any of:
- a 3D cell culture device, preferably a scaffold,
- a 2D cell culture device, preferably a bag, bioreactor, bottle, carrier bead, cell culture dish, film, flask, microscopy slide, multiwell plate, petri dish, migration membrane, perfusion membrane, plate, pouch, roller bottle, spinner flask, tissue slide, tube,
- a microfluidic device, preferably a tube or reactor,
- a biosensor,
- an implantable medical device, preferably an endoprosthetic device, particularly preferred an artificial intervertebral disc, cochlear implant, defibrillator, dental implant, joint replacement device, artificial joint shaft, pacemaker, spine rod, spine screw, stent, surgical pin, surgical plate, surgical rod, surgical wire, surgical screw or suture.

On such devices eukaryotic cells can particularly well attach to the cell attachment surface or cell attachment surfaces of the device. When exposed to a suitable cultivation medium under conditions suitable for cell viability, seeded cells can form colonies and multiply on such surfaces, thereby facilitating cell cultivation and, in particular, expansion of undifferentiated stem cells.

The cell attachment device can be a device for cultivating cells, for example be any device that is suitable for cultivating cells or for handling such cell. In another form devices can for example be any device that is suitable for cultivating cells including handling such cell. A reference herein to a particular "cell culture" device is understood also to mean corresponding tissue culture devices, for example tissue culture vessels.

Examples of devices for cultivating cells include cell culture flasks, cell culture dishes, cell culture plates (e.g. multiwell or microwell plates), cell culture bags, roller bottles and reactors (e.g. bioreactors).

Further examples of devices for cultivating cells which are suitable for handling cell culture containing liquids include tubes, probes, vials, bottles, pipettes and pipette tips, syringes, microscopic slides, microfluidic devices, glass capillaries, biochips, SiO2 wavers or SiO2 arrays.

In a preferred form the device for cultivating cells is selected from cell culture flasks, cell culture dishes, cell culture plates, cell culture bags, reactors, tubes, probes, vials, bottles, pipettes, pipette tips, syringes, microscopic slides, microfluidic devices, glass capillaries, biochips, SiO2 wavers or SiO2 arrays.

In another preferred form, the device for cultivating cells is selected from cell culture flasks, cell culture dishes, bioreactors, test tubes, vials, bottles, pipettes, syringes, microscopic slides, microfluidic devices, glass capillaries, biochips, SiO2 wavers, or SiO2 arrays.

The devices for cultivating cells can be lab scale devices, semi industrial size devices or industrial size devices. Typical industrial sizes of reactors, such as bioreactors are for instance 3 I, 50-200 I or 1000-2000 I. Typical sizes of a well in a cell culture plate is from 0.001 to 10 ml.

According to the invention the polymer conjugate is prepared by a method comprising the steps of
i) providing an amphiphilic copolymer, wherein the amphiphilic polymer comprises a hydrophilic part selected from a polyethylene glycol, and a hydrophobic part, and
ii) conjugating peptides to said copolymer, wherein the peptides comprise, consist essentially of or consist of at least comprising two different integrin binding peptides and at least one positively charged peptide, preferably 3 peptides selected from the group consisting of a linear integrin binding peptide, a cyclic integrin binding peptide and a positively charged peptide.

Preferred peptides and guidelines for choosing particularly preferred combinations thereof are described above. It is a particular advantage that the polymer conjugate of the present invention can be prepared essentially in the moment a cell attachment surface is needed. This is advantageously made possible by the invention, because the copolymer and the peptides can be prepared separately in advance and stored under normal storage conditions (4°C in the dark). Furthermore the preparation of the polymer conjugate does not require specialized equipment, e.g. for surface plasma treatments. Instead, a mere conjugation reaction is sufficient.

The invention thus also provides a method of preparing a cell attachment surface or a cell attachment device of the present invention, comprising the steps of
i) providing an amphiphilic copolymer, wherein the amphiphilic polymer comprises a hydrophilic part selected from a polyethylene glycol, and a hydrophobic part, and
ii) conjugating peptides to said copolymer, wherein the peptides comprise, consist essentially of or consist of at least 3 peptides comprising two different integrin binding peptides and a positively charged peptide, preferably selected from the group consisting of a linear integrin binding peptide, a cyclic integrin binding peptide and a positively charged peptide, and
iii) coating a substrate with the copolymer before, during or after step ii).

Again, preferred peptides and guidelines for choosing particularly preferred combinations thereof are described above. In particular, preferred concentrations of peptides, ratios of cyclic:linear integrin binding peptides:positively charged peptides and surface densities are described above.

The conjugation step can be performed before, during or after coating of the substrate with the copolymer. The method thus advantageously provides a high degree of freedom in the manufacture of cell attachment surfaces and cell attachment devices, in particular cell culture devices.

In one embodiment the conjugation is effected after coating of the surface. Thus, the substrate is first exposed to the polymer to prepare a polymer coating on the substrate. Then, the peptides are added, either sequentially, in rapid succession (i.e. before conjugation has finished) or, more preferred, simultaneously to effect conjugation of the peptides to the coating polymer. It is a particular advantage of the method that only as much polymer has to be used as is required to cover the intended area on the substrate; at least one, preferably all peptides can then be added in amounts to achieve the desired degree of conjugation. Preferably the peptides are provided in a ready to use composition comprising the peptides in the ratios preferred according to the present invention. The peptide composition is preferably applied in surplus to achieve complete conjugation of the polymer bound to the substrate; any excess of peptide composition can then be withdrawn after conjugation. It is a particular advantage that, as described in preferred variants above, the peptides can be conjugated to the polymer without requiring additional chemicals for conjugation. Thus, the excess of peptide composition can be transferred to another surface coated with the copolymer to prepare another cell attachment surface and/or cell attachment device. Thus, the preparation of cell attachment surfaces and devices preferably requires only one opened storage vessel at a time, either the copolymer storage vessel or the peptide storage vessel(s). This is particularly advantageous when preparing such cell attachment surfaces and/or devices in a cleanbench or in a clean room environment, because it increases handling efficiency and reduces the risk of accidental spilling, transfer or contamination of storage vessel contents.

In another embodiment the conjugation is effected during coating of the surface. Thus, polymer and one, two or all peptides are added simultaneously or in rapid succession. In particular it is possible to prepare a conjugation mixture of copolymer and peptides in one cell culture device and transfer as much of the unconjugated or partially conjugated conjugation mixture to one or more further cell culture devices as required for the preparation of the desired cell attachment surfaces of the devices. It is a further advantage of the present invention that the conjugated polymer, as described herein, is capable of coating substrates. Thus, it no particular attention is required in view of the completeness of the conjugation reaction. Instead, a mixture of copolymer and peptides can remain on the substrate to form a polymer conjugate at a first cell attachment surface while a surplus of such mixture - in unconjugated, partially conjugated or fully conjugated form - is transferred for the preparation of a second cell attachment surface.

Thus, in one embodiment the conjugation is effected before coating of the surface. The substrate is then exposed to the conjugated polymer of the present invention. Without being bound to a particular theory it is believed that the polymer conjugate binds to the surface and exposes the conjugated peptides to the cells when such cells are present.

The invention also teaches the use of a polymer conjugate of the present invention for the preparation of a cell attachment surface and/or cell attachment device for the cultivation of adrenal medulla cells, blood cells, bone marrow cells, cardiac muscle cells, muscle cells endocrine cells, epidermal cells, epithelial cells, glandular cells, kidney cells, lung cells, neural cells, osteoblast cells, osteoclast cells, spleen cells, stem cells, thymus cells, degenerate cells, for example tumour cells, cancer stem cells, immortalised cells, and mixtures comprising such cells, preferably stem cells. It is a particular advantage that the present invention provides polymer conjugated and corresponding methods for allowing cell cultivation of such a diverse set of eukaryotic cells types.

Preferably the polymer conjugate of the present invention is used to prepare a cell attachment surface and/or cell attachment device for the cultivation of stem cells, preferably cancer stem cells, oligopotent or pluripotent stem cells, more preferably induced pluripotent stem cells, more preferably human induced pluripotent stem cells. Induced pluripotent stem cells (iPSCs) are cells generated by reprogramming a somatic cell by expressing or inducing expression of a combination of proteins and nucleic acids. iPSCs can be generated using fetal, postnatal, newborn, juvenile, or adult somatic cells. They can be differentiated into non-pluripotent stem cells, e.g hematopoietic stem cells, or into tissue-type cells. As described herein it is a particular advantage that stem cells, in particular induced pluripotent stem cells, can be multiplied (also called "expanded") using standard cell cultivation techniques in the absence of feeder cell layers or the addition of differentiation inhibitor substances in standard cell culture devices in an undifferentiated form.

The invention thus provides a method of eukaryotic cell cultivation, comprising contacting eukaryotic, preferably animal, cells with a polymer conjugate, a cell attachment surface or a cell attachment device according of the present invention.

In view of the aforementioned advantages the cells in such cultivation method according to the invention preferably are selected from the group consisting of adrenal medulla cells, blood cells, bone marrow cells, cardiac muscle cells, endocrine cells, epidermal cells, epithelial cells, glandular cells, kidney cells, lung cells, neural cells, osteoblast cells, osteoclast cells, spleen cells, stem cells, thymus cells, degenerate cells (non-limiting examples are tumour cells, immortalised cells and cancer stem cells) and mixtures comprising such cells, preferably stem cells. Within stem cells, particularly preferred are cancer stem cells, oligopotent or pluripotent stem cells, more preferably induced pluripotent stem cells, more preferably human induced pluripotent stem cells.

Further preferably the cultivation method of the present invention comprises passaging of cell cultures. Such passaging expediently comprises releasing said cells from the polymer conjugate and passaging the cells to another cell attachment surface or cell attachment device of the present invention. Other than providing the required two cell attachment surfaces or devices, respectively, no further precaution for passaging is required. Thus, by applying the polymer conjugate of the present invention, passaging of undifferentiated stem cells or of other cells as described herein is possible using standard and established passaging techniques known in the art.

It is a particular advantage of the present invention that the polymer conjugate of the present invention can be used for maintaining pluripotent stem cells in pluripotent state for at least 5 passages, more preferably 8-40 passages, more preferably 10-25 passages. Furthermore, oligopotent stem cells can be maintained in an oligopotent state for at least 5 passages, more preferably 8-40 passages, more preferably 10-25 passages. Previously great care had to be taken to prevent spontaneous, unwanted differentiation of stem cell cultures during cultivation. This severely limited the availability of oligopotent or pluripotent stem cells for research or other applications, because multiplication of harvested stem cells was not easily possible. The present invention, however, greatly reduces the risk of spontaneous stem cell differentiation without requiring presence of feeder cells or the addition of differentiation inhibitors to stem cell cultures.

The materials, methods and uses described herein are further illustrated in the following examples, which are provided by way of illustration and are not intended to be limiting. It will be appreciated that variations in proportions and alternatives in elements of the components shown will be apparent to those skilled in the art and are within the scope of disclosed forms.

### EXAMPLES

### Example 1 - Preparation of an amphiphilic polymer

371.4 g of methylpolyethylene glycol (average molar mass 500 g/mol), 76.9 g of maleic anhydride and 3.9 g of 4-methoxyphenole were stirred at 130 rpm under nitrogen. The reaction mixture was heated to 90 °C and stirred for another 10 min. Over 4 h 80 g of styrene were added. Simultaneously, 5.6 g of tert-butyl peroxy-2-ethylhexanoate in 28.4 g of the methylpolyethylene glycol were added over 5 h. Afterwards, stirring was continued for another hour at 90 °C. Subsequently, the reaction mixture was heated to 135 °C and stirred for 3 h. The reaction mixture was cooled down to 100 °C and 239 g of demineralized water were added. Water steam distillation was performed over the course of 1 h. The reaction mixture was cooled down to 75 °C and 9.1 g of 5% hydrogen peroxide solution in 46.9 g of demineralized water were added. The reaction mixture was stirred for 30 min at 75 °C and subsequently cooled down to the ambient temperature. The pH of the reaction mixture was adjusted to 8.5 with 96 g of 40% Sodium hydroxide solution. The reaction was stirred for another 2 h at 40 °C, cooled down to the ambient temperature and freeze dried.

### Example 2 - Adding a linker

In order to prepare solution A 1000 mg of freeze dried product of Example 1 were dissolved in 9000 mg of PBS buffer (pH 7.4) and the solution was cooled to 4 °C. 3907 mg of 1-ethyl-3-(3-dimethylaminopropyl)carbodiimide hydrochloride were added and the reaction mixture was stirred at 4 °C for 1 h. Subsequently, 2213 mg of N-hydroxysulfosuccinimide sodium salt were added and stirring was continued for 1 h at 4 °C. To prepare solution B 1295 mg of aminoethyl maleimide were dissolved in 4500 mg of PBS buffer (pH 7.4) and cooled down to 4 °C. Solution A and B were combined at 4 °C and stirred for 16 h, while warming up to the ambient temperature.

The crude product was purified as follows: Ultrafiltration was performed in a stirred (500 rpm) stainless-steel cell (HP7450, Sterlitech, USA) equipped with a 4 kD ultrafiltration membrane (UH004, Microdyn-Nadir, Wiesbaden, Germany) at the ambient temperature. The aqueous solution of F310A after maleimidation with a solid content of 38.4 % was diluted with demineralized water (pH 4, adjusted with HCI) to a final volume of 70 ml. The polymer solution was diafiltrated under a pressure of 8 bar with demineralized water (pH 4, adjusted with HCI) and a diafiltration factor of 7. The sample was freeze dried after ultrafiltration.

### Example 3 - Peptide conjugation

Peptide-polymer conjugates were synthesized as described by the following example: An amount of 0.0034 mmol of product from Example 2 (58967 g/mol) was dissolved in 0.2 L Milli-Q^{®} water, an amount of 0.064 mmol of peptide Ad1c (600 g/mol) was dissolved in a mixture of Milli-Q^{®} water (ϕi = 90 %)/DMSO (ϕi = 5 %) with a total volume of 0.015 L, an amount of 0.032 mmol of peptide Ad12 (1790 g/mol) was dissolved in a mixture of Milli-Q^{®} water (ϕi = 90 %)/DMSO (ϕi = 5 %) with a total volume of 0.047 L and an amount of 0.0064 mmol of peptide PP3 (2241 g/mol) was dissolved 0.0095 L of Milli-Q^{®} water. Peptide solutions were combined and added to the solution of F310A. An additive amount of 0.001 L of DMSO was added to the reaction mixture and the pH value of the reaction mixture was adjusted to 7.5. The reaction mixture was stirred for 2 h at 20 °C and purified via dialysis (MWCO = 25 kDa) against 1 M NaCl for three times and for three times against Milli-Q^{®} water, followed by lyophilization.

### Example 4:

For cultivation of induced pluripotent stem cells (iPSCs) on different polymer-peptide (Ad1 or Ad12) conjugates 6-well plates (non-tissue culture treated plates, Greiner, Germany) were coated with a polymer-peptide conjugate. For this purpose, the polymer of Example 1 comprising the linker as described in Example 2 was dissolved in PBS (1 mg/ml), 1 ml of the solution was added into a well of a 6-well plate and incubated for 30 min at room temperature. Afterwards the polymer solution was removed by pipetting with a 1 ml pipette, and the well was washed once with dH₂O. The peptides Ad1 and Ad12 (0.67 mM) (Ad1c 0.99 mg/ml, Ad12 1.79 mg/ml; Caslo, Denmark) were diluted in dH₂O with 10% dimethyl sulfoxide (DMSO). 1 ml of the peptide solution was added to the polymer treated well plate and incubated for 30 min at room temperature. Thereafter, the wells were washed three times with dH2O. As a control 6-well tissue culture treated plates (TPP, Germany) were coated with Corning Matrigel hESC-qualified Matrix (Matrigel, Corning, Germany). Matrigel were diluted in DMEM-F12 according to manufacturer's instruction (variates from batch to batch). 1 ml of the diluted Matrigel was pipetted into a well and incubated for 1h at room temperature. After removing the solution, the Matrigel wells as well as the polymer-peptide conjugate treated wells were filled with 1 ml mTeSR1 ^{™}-Medium (Stem cell technologies, Germany) and seeded with iPSCs. For cell seeding CRTD3 iPSCs were used. These cells were reprogrammed from human CD34+ cell isolated from peripheral blood from healthy donors (Center for Regenerative Therapies, TU Dresden, Dresden). iPSCs were cultured under standard culture on Matrigel in mTeSR1 ^{™}-Medium at 37°C and 5% CO2. Cells grew in colonies and at 80% confluency iPSCs were split in ratio of 1:6 and seeded in the precoated wells. For cell detachment the cells were washed once with PBS and afterward incubated for 3 min with ReLeSR Passaging Reagent (Stem cell technologies). After 30 s ReLeSR Passaging Reagent was removed and cells were incubated at 37°C. Thereafter cells were diluted in 1 ml mTeSR1 ^{™}-Medium and 166.6 µl of the suspension were added in each well of the precoated (Matrigel, polymer-peptide conjugate) 6-well plates. The plates were incubated at 37°C and 5% CO2.

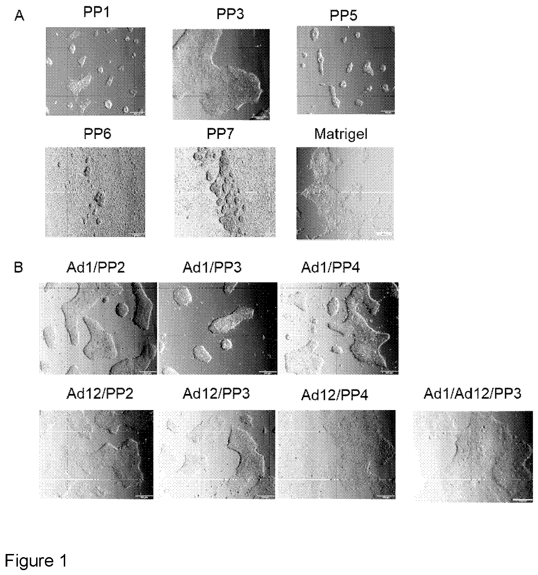

In these approaches only a few adherent colonies on the polymer-peptide conjugated were visible (Figure 1). iPSCs cultured on Ad12 revealed holes in the colonies. This is a sign of differentiation. Furthermore, iPSCs on Ad1 changed their morphology showing flat adherent cells and pluripotency analysis exposed expression of the pluripotency markers SOX2, NANOG, TRA1-60 and OCT3/4 showing that cells still pluripotent.

### Example 5:

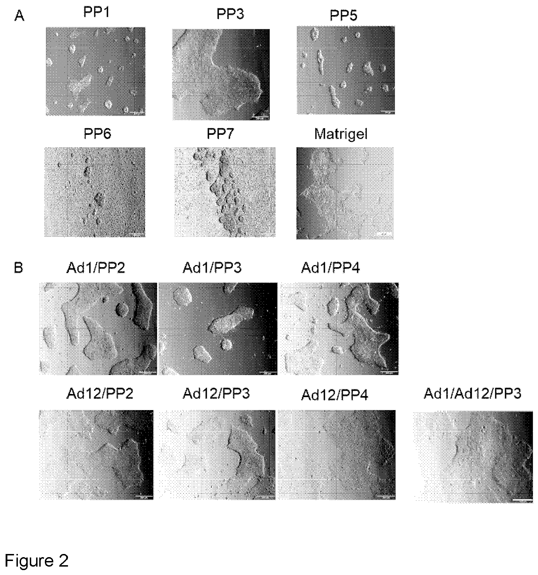

For cultivation of iPSCs on different polymer-peptide (PP1, PP3, PP5, PP6, PP7 and combinations of Ad1/PP2, Ad1/PP3, Ad1/PP4, Ad12/PP2, Ad12/PP3, Ad12/PP4, Ad1/Ad12/PP3) conjugates 6-well plates were coated with the polymer-peptide conjugate. The coating was carried out as described in Example 4. The peptide combinations were mixed in following ratio: Ad1/PP2, Ad1/PP3, Ad1/PP4, Ad12/PP2, Ad12/PP3 and Ad12/PP4 were diluted separately in H2O and mixed in a ratio of 1:1. Peptides in the combination of Ad1/Ad12/PP3 were also diluted in separately in H2O and mixed in a molar ratio of 1(Ad1):1(Ad12):0.2(PP3). As a control one well was coated with Matrigel according to Example 4. Cells were seeded in coated 6-well plates as described in Example 4. Addition of PP1, PP5, PP6, PP7 alone did not allow cell adhesion and spreading of iPSCs showed by small colonies after one day (Figure 2A). Cell adherence was improved on PP3-conjugates showing cell spreading and large colonies of cells. Furthermore, high PP concentration revealed adhesion of cells but cell release for cell splitting was not possible. Combinations of Ad1 with PP2, PP3 or PP4 improved the colony forming but cells' spreading was not comparable to Matrigel (Figure 2B). PP3 in combination with Ad12 supported visibly the cell adhesion and spreading of cells comparable to Matrigel. Nevertheless, cells were able to proliferate only up to passage 1. In contrast, with a combination of Ad1, Ad12 and PP3 undifferentiated iPSCs growth was further enabled until passage 2. It was not possible to maintain iPSCs culture with the described peptide combination PP1, PP3, PP5, PP6, PP7 and Ad1/PP2, Ad1/PP3, Ad1/PP4, Ad12/PP2, Ad12/PP3, Ad12/PP4, Ad1/Ad12/PP3.

### Example 6:

Compared to example 5 Ad1 was replaced by Ad1c. 6-well plates were treated as described in Example 4. The peptides Ad1c:Ad12:PP3 were mixed similar as Ad1:Ad12:PP3 described in Example 5: Cells were seeded on coated 6-well plates described in Example 4. Cells were passaged when a confluency of 80% was reached following the protocol in Example 4. Quantification of cells expressing pluripotency markers was done by flow cytometry. For cell analysis 750 000 cells in 250 µl PBS were incubated with 1.25 µl Tra-1-60 FITC (IgM, Merck Millipore CS204460) and 1.0 µl SSEA-4 PE (IgG3, Merck Millipore CS204438) for 30 min. As a control cells were stained with the appropriate isotype control: Mouse IgG K1 488 (1 µl, BD Bioscience 557721) and Mouse IgG3 PE (+ 1 µl, BD Bioscience 557749) and two samples were stained alone with Tra-1-60 FITC and SSEA-4 PE. After washing the samples with 5% FBS in PBS cells were fixed and analyzed by flow cytometry. For intracellular staining of OCT3/4 and Nanog cells were fixed and permeabilized by using a fixation/permeabilization buffer (Fix/Perm Kit; BD Bioscience 554714) according to manufacturer's instructions. Afterwards, 750 000 cells in 50 µl PBS were stained with 1.25 µl NANOG-647 (BD Bioscience 561300) and 1.25 µl OCT3/4-488 (BD Bioscience 560253) and as a control two separate samples containing the appropriate isotype control each were prepared (1 µl isotype ctrl IgG (H+L) 488 (BD Bioscience 557721) and 1 µl isotype ctrl IgG (H+L) 647 (BD Bioscience 557783)). Furthermore, two samples were stained with OCT3/4 or Nanog alone. All samples were incubated for 30 min, washed two times with BD Perm/Wash buffer and resuspend in 250 µl PBS with 5% FBS prior to flow cytometric analysis.

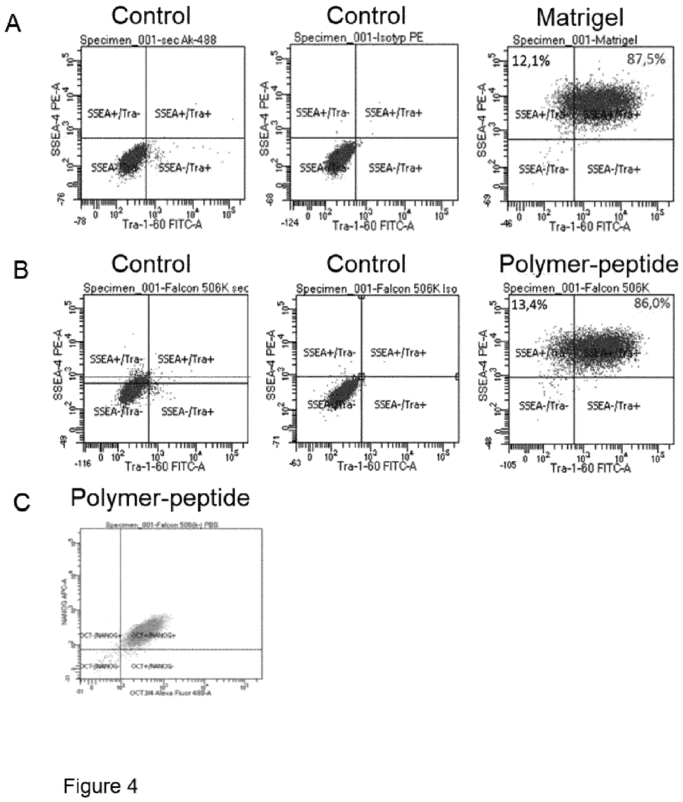

Experiments revealed a significant improvement on cell attachment and growth over a multitude of passages when a combination of the peptides Ad1c:Ad12:PP3 was used (molar ratio of 1(Ad1c):1(Ad12):0.2(PP3)). After first passaging (P1, Figure 4) cells adhered properly and showed a minimal differentiation characterized by long cell protrusions with flat attached cell body. Differentiated, large attached cells were removed by scraping. In P3 cells' morphology was comparable to cells cultured on Matrigel - cell grew in large, dense colonies. Up to P20 big confluent areas of cells in large colonies with distinct borders and barely differentiation (large adhesive cells with long protrusions and large cytoplasm) were visible. Increased cell differentiation was visible from P21 on.

The pluripotency of cells was tested in P12 via immunostaining and revealed high expression of the cell surface marker Oct3/4 (pluripotency marker, data not shown). Small nucleus to cytoplasm ratio, a typical hallmark for IPSCs, appeared for IPSCs cultured on polymer-peptide conjugate. Furthermore, flow cytometry measurements showed a high percentage of Tra 1-60 and SSEA4 double positive cells (86%) which was comparable to cells cultured on Matrigel (87.5%) (Figure 4A/B). After 21 passages of IPSCs on the polymer-peptide conjugate 97.5 % of cells were Oct3/4+ and Nanog+ proving pluripotency of IPSCs (Figure 4C). With these results we could validate a new synthetic polymer which is comparable to non-synthetic Matrigel in stem cell maintenance and proliferation of iPSCs up to high passages.

To show the reproducibility of a culture with the Ad1c:Ad12:PP3 conjugate we restarted another series and analyzed the cells by flow cytometry analysis after passage 12. Cell growth was similar to the results described above: Cell adhesion and proliferation was good; only very few signs of differentiation could be observed, and all cells could be easily detached. A detailed analysis showed that the coating revealed 96.2% double positive OCT3/4+/NANOG+ and 94.9% double TRA-1-60+/SSEA4 cells+. These results were similar to Matrigel with 98.7% OCT3/4+/NANOG+ and 95.7% TRA-1-60+/SSEA4 cells respectively. These results confirm the reproducibility of the culture and coating procedure and the stability of the F310-peptide conjugate.

## Claims

1. Cell attachment polymer conjugate, comprising an amphiphilic polymer and, conjugated thereto, a set of peptides, wherein
the peptides comprise, consist essentially of or consist of at least 3 peptides comprising two different integrin binding peptides and a positively charged peptide, preferably selected from the group consisting of
a) a cyclic integrin binding peptide,
b) a linear integrin binding peptide and
c) a positively charged peptide,
and wherein the polymer comprises hydrophobic moieties and coupling moieties for conjugation of the peptides.

2. Polymer conjugate according to any of the preceding claims, wherein
i) the linear or cyclic integrin binding peptides comprise, consist essentially of or consist of peptides selected from linear or cyclic peptides selected from the group consisting of c(phg-isoDGRX), c(RGDfC), c(RGDfK), c(RGDfV), c(RGDyK), GRGDNP, GRGDTP, RTDLDSLRT, (Ga)NOPO*-c(RGDfK), 44b, A20FMDV2, ATN161, c(FRGDLAFp(NMe)K), Cilengitide, Echistatin, Eptifibatide, F-Galacto-c(RGDfK), Flucilatide, GR144053, JSM6427, Mo/11*, NC100717, RGD-4C, RGD10, sn243, Tirofiban, RGDS, RGDT, RGDV, GRGD, GRGDG, GRGDF, GRGDS, GRGDY, YRGDG, YRGDS, YGRGD, RGDSG, RGDSP, RGDSY, RGDVY, GRGDSP, GRGDSG, GRGDSY, GRGDVY, GRGDSPK, CGRGDSPK, CGRGDSY, RGDfK, YAVTGRGDS, CGGNGEPRGDYRAY, GCGYGRGDSPG, RGDSPASSKP, GRGDSPASSKG, CWGGRGDS, CWGGRGDT, CWGGRGDV, CWGGRGDSG, CWGGRGDSY, CWGGRGDVY, Ad1, Ad1c, Ad12, Ad21 and Ad22,
preferably a linear or cyclic RGD-peptide,
even more preferably Ad1c and Ad12, and/or
ii) the positively charged peptides comprise, consist essentially of or consist of peptides selected from PP2, PP3 and PP4, and/or
iii) peptides differing from the peptides according to i) or ii) by at most 1 amino acid difference, wherein at least one RGD motif in each peptide is conserved.

3. Polymer conjugate according to any of the preceding claims, wherein
the ratio of peptides according to a):b):c) is (0.5-4):1:(0.01-1), more preferably (0.7-3):1:(0.08-0.8), more preferably (1.5-2.5):1:(0.1-0.4).

4. Polymer conjugate according to any of claims 1 to 3, where the amphiphilic polymer comprises a hydrophilic part selected from a polyethylene glycol, and a hydrophobic part.

5. Polymer conjugate according to claim 4, where the hydrophilic part comprises a polyalkylene glycol based on C3-C12 alkylene oxides, or a hydrophobic polymer based on a hydrophobic monomer.

6. Polymer conjugate according to claim 5, where the hydrophobic monomers are aromatic monomers and C1-C18 alkyl (meth)acrylates.

7. Polymer conjugate according to any of claims 1 to 6, where the amphiphilic polymer has a molecular weight of from 5000 to 500,000 g/mol.

8. Cell attachment surface, comprising
a substrate coated with the polymer conjugate according to any of the preceding claims.

9. Cell attachment device, comprising a cell attachment surface according to claim 8.

10. Method of preparing a polymer conjugate according to any of claims 1-7, comprising the steps of
i) providing a copolymer, wherein the amphiphilic polymer comprises a hydrophilic part selected from a polyethylene glycol, and a hydrophobic part, and
ii) conjugating peptides to said copolymer, wherein the peptides comprise, consist essentially of or consist of at least comprising two different integrin binding peptides and at least one positively charged peptide, preferably at least 3 peptides selected from the group consisting of a linear integrin binding peptide, a cyclic integrin binding peptide and a positively charged peptide.

11. Method of preparing a cell attachment surface according to claim 8 or a cell attachment device according to claim 9, comprising the steps of
i) providing a copolymer, wherein the amphiphilic polymer comprises a hydrophilic part selected from a polyethylene glycol, and a hydrophobic part, and
ii) conjugating peptides to said copolymer, wherein the peptides comprise, consist essentially of or consist of at least 3 peptides comprising two different integrin binding peptides and a positively charged peptide, preferably selected from the group consisting of a linear integrin binding peptide, a cyclic integrin binding peptide and a positively charged peptide, and
iii) coating a substrate with the copolymer before, during or after step ii).

12. Use of a polymer conjugate according to any of claims 1-7 for the preparation of a cell attachment surface and/or cell attachment device for the cultivation of adrenal medulla cells, blood cells, bone marrow cells, cardiac muscle cells, muscle cells, endocrine cells, epidermal cells, epithelial cells, glandular cells, kidney cells, lung cells, neural cells, osteoblast cells, osteoclast cells, spleen cells, stem cells, thymus cells, degenerate cells and mixtures comprising such cells, preferably stem cells.

13. Method of eukaryotic cell cultivation, comprising
contacting eukaryotic cells with a polymer conjugate according to any of claims 1-7, a cell attachment surface according to claim 8 or a cell attachment device according to claim 9.

14. Cultivation method according to claim 13, wherein
the cells are selected from the group consisting of adrenal medulla cells, blood cells, bone marrow cells, cardiac muscle cells, muscle cells, endocrine cells, epidermal cells, epithelial cells, glandular cells, kidney cells, lung cells, neural cells, osteoblast cells, osteoclast cells, spleen cells, stem cells, thymus cells, degenerate cells and mixtures comprising such cells, preferably somatic, oligopotent or pluripotent stem cells.

15. Cultivation method according to claim 14, further comprising the step of releasing said cells from the polymer conjugate and passaging the cells to another cell attachment surface according to claim 8 or cell attachment device according to claim 9.

16. Use of a polymer conjugate according to any of claims 1-7 for maintaining stem cells in a pluripotent state, preferably in the absence of differentiation inhibitors, for at least 5 passages, more preferably 8-40 passages, more preferably 10-25 passages.
